# EUROPEAN PATENT APPLICATION

(11) **EP 0 631 131 A1**
(43) Date of publication of application: **28.12.1994**
(21) Application number: 94109181.1
(22) Date of filing: 15.06.1994
(51) Int. Cl.: G01N 27/411, G01N 33/20

(54) **Electrochemical sensor for determining sulfur content in molten ferrous baths**

(30) Priority: 21.06.1993 IT RM930396
(71) Applicant: ILVA LAMINATI PIANI S.p.A., I-00185 Roma (IT)
(72) Inventor: Granati, Paolo, I-00168 Roma (IT)
(74) Representative: Gervasi, Gemma, Dr.

(57) **Abstract**

A careful choice of materials and of their coupling allows to obtain an electrochemical sensor capable of precisely and reliably determining the sulfur content of molten iron-based metal baths. Said sensor can also work continuously for periods of time comparable to the processing ones for pig-iron or steel.

## Description

### FIELD OF INVENTION

The present invention relates to an electrochemical sensor for sulfur determination in molten iron-based baths and, more particularly, to a Ca-concentration cell capable to work for tens of minutes at temperatures ranging from 1250 to 1700 °C, for sulfur determination in molten pig-iron or steel baths.

### STATE OF THE ART

In the production of pig-iron and steel, owing to the high investment costs and to the low market value of the products, a continuous effort is being made to improve the quality of the end-products and to enhance the productivity of the plants. Thus, great care is used for expanding the utilization of continuous production processes and their automation as well as for accurately controlling said processes and the quality of relevant products.

Clearly, the chemical composition of the products is a quality essential factor. A number of quality control techniques have been developped, all based on the laboratory analysis of samples coming from the steel plant or from the blast-furnace cast house. Only in some cases said analysis techniques did evolve in such a way to be carried out in real time, and even not in the laboratory but at the very metal working place.

However, we are still far from being able to control in real time the majority of the chemical elements; in particular, for the sulfur, which is a very important element for the brittleness of steel and pig-iron, its analysis still requires, from the sampling until the analysis result is obtained, an execution time confrontable with the processing time of the metal.

This obviously permits the quality check of the obtained product, but in the majority of cases prevents an intervention during the production process to optimize the product composition.

Though for the sulfur analysis several methods and devices capable to work up to 700-800 °C have been introduced in the chemical and in the petrochemical fields, as well as in the field of non-ferrous metals, in the field of pig-iron and steel production the very high temperatures reached by the baths, from 1250-1300 °C up to 1600-1700 °C, did prevent up to now the development of suitable sensors, which are also able to work continuously immersed in the molten bath for several minutes.

### SUMMARY OF THE INVENTION

The object of present invention is to provide an electrochemical sensor for quickly determining the sulfur content of iron-based molten metal baths.

Another object of the present invention is to provide a calcium concentration cell, for the quick continuous determination of the sulfur content of molten iron-based metal baths.

### DETAILED DESCRIPTION OF THE INVENTION

The calcium-concentration cell according to the present invention is characterized by the combination of: (i) a solid electrolyte consisting in Ca-β alumina, in the form of a sintered pellet having a first and a second face parallel to one another; (ii) a reference electrode having known calcium activity, consisting of a layer of a mixture of a metal and its oxide, which is in contact with the first face of said pellet of solid electrolyte; (iii) a CaS pellet, in contact with the second face of said solid electrolyte pellet and with the molten metal bath; (iv) a case of non-conducting refractory.

Preferably, in the reference electrode the metal is niobium.

The cell is completed by an alumina layer, in contact with said reference electrode, and bearing a superimposed carbon powder layer, preferably graphite (to prevent oxygen entering the cell), and by a platinum wire connected to said pellet of solid electrolyte. An embodiment, prepared with the aim of executing preliminary experiments on cell reliability, comprises an alumina tubular body case, bearing welded inside in proximity of one of its ends a CaS disc, having a diametre equal to the internal diametre of the case and prepared by sintering analytical grade calcium sulphide . Within the so defined cavity, in contact with said CaS disc and welded to the case, a Ca-β alumina pellet is placed, whereupon a layer of a Nb+NbO mixture, a layer of alumina and a layer of graphite are sequentially placed, in contact with one another. A platinum wire is welded to the Ca-β alumina pellet and passes through all the superimposed layers, to carry the cell signal to the meter.

The working principle of the cell is the following: at the cell working temperature, the couple niobium/niobium oxide is in equilibrium with a precise and known oxigen partial pressure, which in turn is in equilibrium with the calcium in the Ca-β alumina, according to the following reactions:

NbO₍ₛ₎ = Nb ₍ₛ₎ + 1/2 O_{2(g)}

Ca²⁺ + 1/2 O_{2(g)} = CaO(Ca-βAl₂O₃) + 2e

thus determining a well defined and known activity for the calcium. At the other side of the cell, the calcium sulphide is in equilibrium with the molten bath according to the reaction:

CaS₍ₛ₎ = Ca²⁺ + S + 2e

In this reaction, the sulfur activity, which corresponds to the one of the sulfur in the bath, is related to a precise quantity of calcium, so that by measuring the electromotive force of said calcium-concentration cell, a direct measurement of the sulfur content of the bath is obtained.

### EXAMPLE

To check the good functioning of the cell according to present invention, its accuracy and the reproducibility of the measurements, as well as the feasibility of continuous measurements, a carbon saturated pure iron bath was prepared to which known quantity of sulfur, as FeS, were added.

The bath temperature was mantained at around 1600 °C.

Before and after each addition of iron sulphide, the bath was omogeneized by agitation, and samples were taken for reference analysis, to be performed with the conventional spectrochemical method.

In the following table the sulfur content values are reported, as spectrochemically measured (Sa) in the first line and as measured with the cell according to present invention (Sd) in the second line, while in the third line the mV measurements of the electromotive force of the cell are reported.

**Table 1**

| | | | | | | | | |
|---|---|---|---|---|---|---|---|---|
| Sa, ppm | 8.43 | 11.42 | 19.56 | 26.06 | 51.93 | 89.4 | 126.95 | 152.52 |
| Sd, ppm | 7.9 | 10.3 | 19.5 | 27.5 | 50.2 | 91.2 | 118.4 | 140.3 |
| FEM, mV | -65 | -68 | -68.8 | -72 | -77 | -84 | -90 | -96 |

As it is apparent from the reported data, there is a very good agreement between the two measurements.

The measurements were repeated many times, both in the same bath (measurements repeated during 10 minutes) and in different baths, taking care to add about the same sulfur amount; the reproducibility obtained was very high, with a reliability level over 94%.

The probe according to present invention proved to be able to continuously work immersed in a molten bath at 1600 °C for periods of over an hour, in any case sufficient in any kind of treatment or of pig-iron or steel processing.

## Claims

1. Ca-concentration cell for determining the sulfur content in molten iron-based metal baths, comprising the following active parts:
(i) a solid electrolyte consisting of Ca(β-alumina), in the form of a sintered pellet having a first and a second face parallel to one another, (ii) a reference electrode having known calcium activity, made by a layer of a mixture of a metal and its oxide, which is in contact with the first face of said pellet of solid electrolyte, (iii) a CaS pellet, in contact with the second face of said solid electrolyte pellet and with the molten metal bath, said active parts being enclosed in a refractory non-conducting case and being interfaced with the metal bath through said CaS pellet.

2. Ca-concentration cell according to claim 1, characterized in that the metal in the reference electrode is niobium.

3. Ca-concentration cell according to claim 1, characterized in that the reference electrode is protected against possible leakages of oxigen within the cell by an alumina layer which is in turn in contact with a carbon layer.
